Europäisches Patentamt

(19) European Patent Office

Office européen des brevets

(11) Numéro de publication: **0 196 946 B1**

(12) **FASCICULE DE BREVET EUROPEEN**

(45) Date de publication du fascicule du brevet:
23.05:90

(51) Int. Cl.⁵: **A61F 2/34**, A61F 2/30, B29C 43/16, B29C 67/24

(21) Numéro de dépôt: **86400487.4**

(22) Date de dépôt: **07.03.86**

(54) **Pièce en polyoléfine de haut poids moléculaire notamment pour prothèse articulaire et son procédé de fabrication par forgeage en matrice fermée.**

(30) Priorité: **12.03.85 FR 8503591**

(43) Date de publication de la demande:
**08.10.86 Bulletin 86/41**

(45) Mention de la délivrance du brevet:
**23.05.90 Bulletin 90/21**

(84) Etats contractants désignés:
**BE CH DE GB IT LI NL**

(56) Documents cités:
**FR-A- 2 338 690
US-A- 4 110 391
US-A- 4 195 368
US-A- 4 205 400
US-A- 4 209 480**

**MODERN PLASTICS INTERNATIONAL,
vol. 13, no. 2, février 1983, Lausanne, CH;
"Self-reinforced polyethylene has potential for
high-precision parts"
MODERN PLASTICS INTERNATIONAL,
octobre 1981, pages 38,39; G. MENGES et al.:
"Self-reinforcing plastics: a new approach to
high-performance resins"**

(73) Titulaire: **Société anonyme: COMPAGNIE ORIS
INDUSTRIE, 33, rue de la Fédération BP 510,
F-75752 Paris Cédex(FR)**
Titulaire: **ASSOCIATION POUR LA RECHERCHE ET LE
DEVELOPPEMENT DES METHODES ET PROCESSUS
INDUSTRIELS (ARMINES), 60, Boulevard Saint-Michel,
F-75272 Paris Cédex 06(FR)**

(72) Inventeur: **Gaussens, Gilbert, 11, rue Jean Brunet,
F-92190 Meudon(FR)**
Inventeur: **Haudin, Jean Marc, Résidence Turquoise
Route de Saint Jean, F-06600 Antibes(FR)**
Inventeur: **Monasse, Bernard, Le Val de la Mer Chemin
Saint Jean, F-06600 Antibes(FR)**

(74) Mandataire: **Pottier, Pierre Société BREVATOME et al,
25, Rue de Ponthieu, F-75008 Paris(FR)**

ACTORUM AG

## Description

La présente invention a pour objet une pièce en polyoléfine de haut poids moléculaire, notamment pour prothèse articulaire, résistant au frottement et au fluage, et un procédé de fabrication de ladite pièce par forgeage en matrice fermée.

De façon plus précise, elle concerne des pièces pour prothèse articulaire en polyoléfine de haut poids moléculaire, dans lesquelles les chaînes de la polyoléfine présentent une orientation particulière qui est obtenue lors du forgeage des pièces en matrice fermée et qui permet d'améliorer la résistance au fluage et à l'usure de ces pièces.

Les prothèses articulaires telles que les prothèses totales de la hanche sont constituées généralement de deux pièces comportant une partie hémisphérique, frottant l'une contre l'autre. Ces deux pièces sont :

- d'une part, une prothèse fémorale avec une queue et une tête solidaires ou non, la queue étant réalisée en alliage métallique, par exemple en acier inoxydable, en alliage cobalt-chrome-molybdène ou en alliage de titane, et la tête étant métallique ou céramique, par exemple en alumine frittée ; et
- d'autre part, une prothèse cotyloïdienne appelée "cotyle" fixée sur le bassin à l'aide d'un ciment en polymère, par exemple en polyméthacrylate de méthyle. Le cotyle peut être constitué d'une matière céramique telle que l'alumine frittée, mais il est, la plupart du temps, réalisé en polyéthylène de très haut poids moléculaire.

En effet, les céramiques sont des matériaux fragiles, ce qui limite actuellement leur développement dans ce type de prothèse. De ce fait, la majorité des prothèses actuelles comportent un cotyle en polyéthylène de haut poids moléculaire, et leur durée de vie est estimée à 12 ans environ. Cette dernière ne semble pas limitée par la biocompatibilité du matériau, mais plutôt par des phénomènes de fluage et d'usure du cotyle.

Aussi, des recherches sont poursuivies actuellement pour tenter d'augmenter la longévité des prothèses articulaires. Dans ce but, on peut envisager, soit de changer les matériaux constituant la prothèse, soit de tenter d'améliorer les propriétés mécaniques des prothèses existantes.

La deuxième solution parait plus intéressante car elle évite d'avoir à reprendre l'étude de la prothèse et d'avoir à tester la biocompatibilité des matériaux sur une période suffisamment longue ; en effet, la plupart des rejets dus à une mauvaise biocompatibilité n'apparaissent qu'après plusieurs années d'implantation.

La présente invention a précisément pour objet une pièce en polyoléfine de haut poids moléculaire, notamment pour prothèse articulaire, dont la structure a été modifiée par forgeage en matrice fermée, de façon à améliorer les propriétés mécaniques de la pièce, notamment sa résistance au fluage et à l'usure.

La réalisation de pièces en matière thermoplastique par forgeage en matrice fermée, est une technique bien connue qui a été mise en oeuvre depuis plusieurs années pour fabriquer des pièces en polypropylène et en polyéthylène de haute densité. Cependant, jusqu'à présent, les recherches effectuées sur le forgeage de prothèses en matériaux thermoplastiques n'ont pas permis d'obtenir, grâce au choix des conditions de forgeage, une amélioration possible des propriétés mécaniques des pièces.

En effet, jusqu'à présent, le choix des conditions de forgeage a surtout été étudié en vue d'obtenir des pièces exemptes de défaut à partir de matières plastiques difficiles à forger, telles que les polyacétals, qui présentent une faible ductilité. Ceci est décrit en particulier par K.M. KULKARNI dans le brevet américain US-A 3 825 648, qui étudie l'influence de certaines conditions de forgeage sur la qualité des pièces obtenues et en déduit que l'un des paramètres importants est la vitesse de forgeage qui ne doit pas excéder une valeur reliée de façon étroite à un coefficient de complexité qui tient surtout compte de la forme de la pièce à réaliser. Ainsi, dans ce coefficient, la notion d'hétérogénéité de la déformation n'intervient pas et il n'est pas prévu d'obtenir une amélioration des propriétés mécaniques de la pièce en favorisant certaines déformations locales.

Toutefois, d'autres auteurs tels que Paul Raymond SMITH "Solid-phase forming polymers" Mars 1979, University of Leeds, Thèse pour l'obtention du titre de "Docteur en philosophie", ont envisagé d'améliorer les propriétés mécaniques de prothèses en polymère thermoplastique tel que le polyéthylène, par le choix de certaines conditions de forgeage. Ainsi, SMITH a trouvé que certaines conditions de forgeage permettent d'améliorer la dureté des pièces, mais à la suite de ses expérimentations, il n'a pas été possible d'obtenir des pièces présentant des résistances à l'usure et au fluage améliorées par rapport à celles de pièces usinées.

Or, on sait que le fluage est un phénomène très important dans l'estimation de la résistance à long terme des prothèses, car c'est le phénomène principal d'évolution du cotyle.

On connaît aussi par le brevet US-A 4 110 391 un procédé de préparation d'articles moulés en polyoléfine de haut poids moléculaire, utilisable pour la fabrication de prothèses, qui consiste à introduire dans un moule de la poudre de polyéthylène ou d'un copolymère d'éthylène, à fermer le moule, à chauffeur celui-ci en atmosphère inerte à une température de 150 à 250°C, de préférence de 190 à 210°C, à comprimer la polyoléfine dans le moule pendant au moins 2 minutes à une pression de 2,5 à 25N/mm², puis à comprimer l'ensemble pendant au moins une minute à une pression de 40 à 100N/mm².

La présente invention a précisément pour objet un procédé de préparation d'une pièce en polyoléfine de haut poids moléculaire, notamment pour prothèse articulaire, présentant des propriétés mécaniques améliorées, notamment en ce qui concerne sa résistance à l'usure et au fluage, et un procédé de fabrication de ladite pièce par forgeage en matrice fermée.

Elle concerne également la pièce en polyoléfine de haut poids moléculaire, obtenue par ce procédé qui comporte une surface destinée à être en contact avec une autre pièce, dite surface de frottement. Par ce procédé, la structure de la polyoléfine est telle que les chaînes de la polyoléfine sont orientées parallèlement à ladite surface de frottement, au moins dans la zone de ladite pièce affleurant pratiquement ladite surface de frottement, de façon à obtenir une résistance au fluage au bout de 48 heures équivalant à une déformation par fluage d'au plus 16% dans l'essai de fluage selon la norme ASTM D 695–80.

On précise que, selon l'invention, on entend par polyoléfine de haut poids moléculaire une polyoléfine telle qu'une ébauche réalisée en cette polyoléfine et n'ayant pas subi de déformation préalable conserve sa forme initiale lorsqu'elle est portée à une température allant de la température de fusion Tf de la polyoléfine jusqu'à environ 10°C au-dessus de cette température de fusion Tf.

Généralement, la polyoléfine est du polyéthylène.

Selon un mode préféré de réalisation de l'invention, la pièce est une pièce pour prothèse articulaire destinée par exemple à une prothèse de la hanche, du genou ou de l'épaule.

Cette structure particulière de la pièce de l'invention, est obtenue par le procédé de l'invention qui fait appel au forgeage en matrice fermée, grâce au choix de certaines conditions de forgeage, qui permettent de maîtriser les taux de déformation locale de la polyoléfine et d'obtenir ainsi cette orientation des chaînes de la polyoléfine au moins dans la zone de la pièce située en dessous ou affleurant la surface de frottement.

Selon l'invention, le procédé de fabrication d'une pièce en polyoléfine comportant une surface destinée à être en contact avec une autre pièce, dite surface de frottement, se caractérise en ce qu'il comprend les étapes suivantes:

a) porter à la température de forgeage une ébauche de ladite polyoléfine, dans laquelle le rapport longueur de l'ébauche par rapport à la dimension transversale de l'ébauche est d'environ 0,6 à environ 1,7, la température de forgeage étant située dans la gamme de températures allant d'environ 20°C en dessous de la température de fusion Tf de ladite polyoléfine à environ 10°C au-dessus de cette température de fusion Tf,

b) disposer l'ébauche portée à ladite température de forgeage dans une matrice de forgeage susceptible d'être obturée par un poinçon déplaçable en translation dans ladite matrice dans le sens de la hauteur de l'ébauche, le poinçon déplaçable et/ou la matrice étant à une température allant de la température ambiante à la température de forgeage,

c) appliquer au poinçon une pression supérieure à la contrainte d'écoulement de ladite polyoléfine à ladite température de forgeage et déplacer ledit poinçon jusqu'à une position finale pour forger ladite ébauche dans ladite matrice, avec un taux de réduction de 0,4 à 0,8, et une vitesse de forgeage de 10 à 150 cm par minute,

d) maintenir le poinçon en position finale pendant une période allant d'environ 90 à environ 150 secondes pour obtenir un retrait homogène et un bon contrôle dimensionnel de la pièce finale, et

e) extraire la pièce forgée de la matrice.

Le choix, selon l'invention, des conditions de forgeage précitées permet d'obtenir le taux de déformation voulu, qui constitue le facteur essentiel pour l'amélioration des propriétés mécaniques de la pièce forgée. Ainsi, la géométrie de l'ébauche qui est définie par le rapport longueur de l'ébauche/dimension transversale de l'ébauche est un facteur primordial qui intervient pour définir le taux de déformation local de la matière. Selon l'invention, on utilise une ébauche ayant un rapport longueur/dimension transversale de 0,6 à 1,7, car les déformations sont plus fortes lorsque la longueur est importante par rapport à la dimension transversale de l'ébauche. Toutefois, on ne peut dépasser la valeur de 1,7, car au-delà de cette valeur, on peut obtenir certains défauts, par exemple une mauvaise sphéricité de la surface de frottement dans le cas d'un cotyle de prothèse de la hanche. De plus, pour des valeurs importantes du rapport longueur/dimension transversale, il risque de se produire un flambage de l'ébauche. A des valeurs inférieures à 0,6, le taux de déformation n'est pas suffisant pour obtenir une orientation des chaînes de la polyoléfine suffisante pour améliorer la résistance au fluage.

Pour obtenir le taux de déformation voulu dans de bonnes conditions, les dimensions de l'ébauche doivent être adaptées aux dimensions de la pièce à réaliser. Il est préférable en particulier que la longueur de l'ébauche soit telle que le taux de réduction obtenu par forgeage soit de 0,4 à 0,8. Le taux de réduction est défini par la formule :

$$\frac{H_i - H_f}{H_i}$$

dans laquelle $H_i$ est la longueur de l'ébauche et $H_f$ est la longueur de la pièce forgée ; dans le cas des prothèses de la hanche, $H_f$ est l'épaisseur du cotyle.

D'autres paramètres tels que la température de forgeage, la température de la matrice et la vitesse de forgeage influent également sur les taux de déformation locaux à l'intérieur de la pièce. Ainsi, la température de forgeage à laquelle on porte l'ébauche, constitue un paramètre important, car c'est le principal facteur qui influe sur le comportement de la polyoléfine. Selon l'invention, on choisit cette température de forgeage dans la gamme allant de 20°C en dessous de la température de fusion Tf de ladite polyoléfine jusqu'à 10°C au-dessus de cette température de fusion Tf.

On précise que la température de fusion Tf de la polyoléfine est déterminée par calorimétrie et qu'elle correspond au maximum de la vitesse de transformation enregistrée en calorimétrie. La fusion d'une polyoléfine change le comportement mécanique de celle-ci qui passe de viscoélastoplastique à viscoélastique. Ce comportement viscoélastique est observé à l'état fondu et la forte viscosité de la polyoléfine de haut poids moléculaire permet sa transformation par forgeage au-dessus de la température de fusion ; cependant, l'orientation de la matière y est moindre que lors d'un forgeage à l'état solide. A basse température, c'est-à-dire à plus de 20°C en-dessous de la température de fusion, la polyoléfine est fragile, ce qui fait apparaître des ruptures au sein de celle-ci. La meilleure température de forgeage se situe donc dans le domaine de températures qui correspond à la fusion de la polyoléfine et la réalisation du forgeage à une température correspondant sensiblement à la fin de la fusion donne les meilleurs résultats, c'est-à-dire une bonne orientation des chaînes de la polyoléfine et peu de défauts résiduels.

La température de forgeage a par ailleurs de nombreuses conséquences sur les pièces forgées. En effet, la localisation de la déformation dépend fortement de la température de l'ébauche, notamment lorsqu'on utilise un poinçon à la température ambiante. Dans ces conditions, il apparaît toujours une zone morte et la déformation est reportée vers des zones plus internes de la pièce. Lorsque la température de l'ébauche est faible, la déformation se localise surtout à proximité de la zone morte, laissant les zones les plus internes moins orientées, alors qu'une température plus élevée répartit la déformation sur une plus grande épaisseur. Dans ce dernier cas, le taux de déformation local maximum est toutefois moins élevé, mais il peut être suffisant. La température de forgeage exerce également un effet sur l'apparition et la localisation des défauts dans la pièce forgée. En effet, ces défauts sont créés lors du retrait du poinçon par le retour élastique du polymère suivant la théorie de Griffith. Lors de la suppression de la charge, le retour élastique du polymère met les extrémités des fissures pré-existantes en traction et provoque leur extension dans des directions parallèles à la surface. Cet effet est d'autant plus important que le retrait élastique est grand, donc à basse température.

Aussi une température de forgeage relativement basse, influe à deux niveaux distincts sur l'apparition des défauts :

1) en localisant la déformation dans la pièce, ce qui conduit à une augmentation du niveau de déformation local maximal, et
2) en augmentant le retour élastique du polymère, ce qui conduit à un agrandissement des fissures.

Dans le cas du forgeage de pièces ayant la forme de cotyles, les deux mécanismes de fissuration provoquent le développement de trous dans des plans parallèles à la surface interne du cotyle et les zones de plus forte déformation sont essentiellement localisées au fond du cotyle. De ce fait, l'apparition et le développement de ces trous provoquent une décohésion du polymère et une relaxation partielle des contraintes internes. Ces deux effets conduisent à un aplatissement du fond du cotyle où les trous sont localisés, et à des défauts de sphéricité. Cependant, cet effet qui est très important à des températures relativement basses devient négligeable à température élevée.

Aussi, la fusion partielle de la polyoléfine est un paramètre majeur dans le procédé de forgeage, car elle a une grande influence sur les propriétés et les défauts de la pièce forgée. Dans le procédé de l'invention, on porte l'ébauche à la température choisie pour le forgeage par chauffage dans une étuve, de préférence ventilée par de l'air, et la durée du chauffage est de préférence de 2 à 5h pour obtenir une bonne homogénéité de température dans l'ébauche.

Les températures de la matrice et/ou du poinçon peuvent également jouer un rôle sur l'orientation des chaînes de la polyoléfine, lors du forgeage. En effet, si l'on utilise un poinçon froid (à la température ambiante), on abaisse localement la température de la polyoléfine en augmentant ainsi son module d'élasticité et sa contrainte d'écoulement. La zone refroidie n'est plus déformée et se transforme ainsi en zone morte. La déformation est reportée au-delà de cette zone morte et le taux de déformation des grains et donc l'orientation des chaînes de la polyoléfine sont augmentés. Ainsi, le choix de la température du poinçon et du temps de contact du poinçon avec l'ébauche qui dépend de la vitesse de forgeage, permettent de contrôler l'épaisseur de la zone morte. En revanche, une température de poinçon supérieure permet d'éliminer la zone morte et, dans ce cas, la déformation est maximale à la surface interne de la pièce forgée à laquelle sont associés des défauts orientés dans la direction de plus grande extension du polymère, parallèlement à la surface.

Selon l'invention, les températures du poinçon et/ou de la matrice peuvent aller de la température ambiante, par exemple d'environ 20°C, à la température de forgeage et l'on choisit ces températures en fonction de la zone de la pièce que l'on veut modifier par orientation des chaînes de la polyoléfine. Par ailleurs, le poinçon et la matrice peuvent être à des températures différentes. Lorsque l'on utilise un poinçon maintenu à une température inférieure à la température de forgeage, la vitesse de forgeage, c'est-à-dire la vitesse de déplacement du poinçon, qui détermine le temps de contact entre le poinçon et l'ébauche durant la déformation, constitue également un paramètre important. En effet, celui-ci détermine également l'épaisseur de la zone morte et de ce fait, les zones de la pièce où les chaînes de polymère seront orientées.

La vitesse de forgeage exerce également une influence sur la rhéologie du polymère, mais ce phénomène est sans doute moins important. Selon l'invention, on peut utiliser des vitesses de forgeage allant de 10 à 150 cm/min.

Par ailleurs, cette vitesse peut varier, si on le désire, au cours du forgeage, en restant toutefois de préférence dans les limites indiquées ci-dessus.

Le temps de maintien du poinçon en position finale et la charge qui lui est appliquée constituent aussi des paramètres importants du procédé de forgeage. En effet, les polyoléfines ont un comportement viscoélastoplastique et lorsqu'une charge constante leur est appliquée, les contraintes internes à la polyoléfine peuvent se relaxer partiellement. Ces contraintes sont responsables du retour élastique lors du retrait de la charge. Même dans une géométrie simple, la répartition des contraintes et l'orientation des chaînes de polyoléfine sont complexes. Le retour élastique est alors difficilement prévisible.

Dans le cas d'une cavité cylindrique, lorsqu'on fait varier le temps de maintien du poinçon en position finale, sous charge maximale, on obtient des variations dimensionnelles qui peuvent donner une géométrie du type diabolo lorsque ce temps de maintien est faible, ou une déformation en tonneau lorsque le temps de maintien est long. Généralement, le temps de maintien sous charge maximale est d'au moins 90 secondes et il ne doit pas dépasser environ 150 s. De préférence, pour obtenir un retrait homogène de la polyoléfine et un bon contrôle dimensionnel des pièces forgées, le temps de maintien sous charge maximale, est d'environ 2 minutes.

La charge maximale c'est-à-dire la pression appliquée au poinçon doit être supérieure à la charge nécessaire pour remplir totalement la matrice. Cette valeur minimale correspond à la contrainte d'écoulement de la polyoléfine à la température de forgeage. Dans le cas du polyéthylène, cette contrainte d'écoulement à 135°C est de 2,5 MPa, et on peut utiliser pour le forgeage une pression de 10 à 80 MPa.

Le procédé de l'invention peut être utilisé en particulier pour la réalisation de cotyles pour prothèses de la hanche. Dans ce cas, le poinçon déplaçable peut être un poinçon hémisphérique, associé de préférence à une matrice cylindrique ayant un fond hémisphérique en regard du poinçon.

Généralement, la matrice et le poinçon sont réalisés en acier ou en alliage léger.

D'autres caractéristique et avantage de l'invention apparaîtront mieux à la lecture de la description qui suit, donnée bien entendu à titre illustratif et non limitatif en référence au dessin annexé sur lequel :

- la figure 1 est un diagramme de détermination de la température de fusion du polyéthylène utilisé,
- les figures 2a, 2b et 2c représentent de façon schématique en coupe verticale, un dispositif de forgeage en matrice fermée utilisé pour la préparation de cotyles pour prothèses de la hanche, et
- la figure 3 représente de façon schématique une autre configuration de forgeage en matrice fermée.

On décrit ci-après un exemple de mise en oeuvre du procédé de l'invention pour la réalisation de cotyles en polyéthylène de haut poids moléculaire pour prothèses de la hanche présentant les dimensions suivantes :

- diamètre externe maximal : 49,7 ± 0,2 mm,
- diamètre interne :22,7 ± 0,1 mm,
- épaisseur :13,5 ± 0,2 mm.

On prélève l'ébauche qui sera soumise au forgeage dans une plaque de polyéthylène obtenue par compression et frittage d'une poudre de polyéthylène, dans les conditions suivantes :

- compactage sous une pression de 10 MPa à la température ambiante,
- montée en température jusqu'à 200°C sous une pression de 1 MPa,
- maintien à 200°C, puis refroidissement jusqu'à la température ambiante.

Le polyéthylène utilisé est du polyéthylène commercialisé sous la référence HD 1000 par ERTA et l'ébauche prélevée est de forme cylindrique de section circulaire et elle a une longueur de 46 mm et un diamètre de 28 mm. Le polyéthylène HD 1000 présente les caractéristiques suivantes :

- masse volumique : 0,94 g/cm³,
- point de fusion : 135-138°C,
- dureté Shore : D 65
- allongement à la rupture : >100% avec une vitesse de traction de 125 mm/min.
- module d'élasticité en traction : 650 MPa.

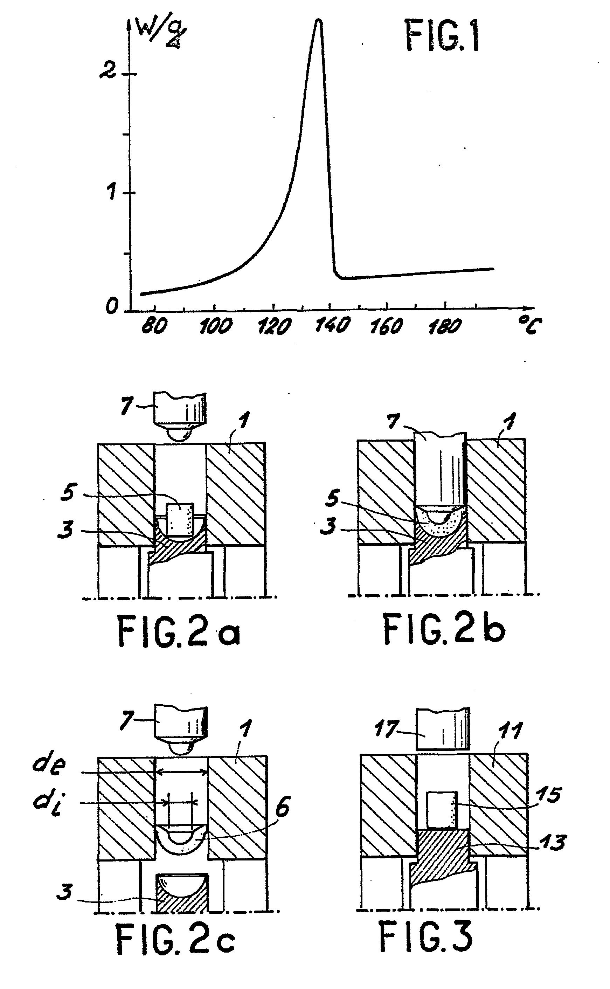

Pour choisir la température de forgeage, on détermine tout d'abord le point de fusion de ce polyéthylène en utilisant un analyseur calorimétrique différentiel à compensation de puissance du type PERKIN EL-MER DSC 2B. Les résultats obtenus sont donnés sur la figure 1 qui est un diagramme représentant l'évolution de la chaleur massique en fonction de la température. Le pic de fusion se superpose à la courbe donnant l'évolution de la chaleur massique et l'on constate que la température de fusion qui correspond au maximum de la transformation enregistrée en calorimétrie est de 136°C.

Pour le forgeage, on utilise une presse hydraulique dont la charge maximale est de 10 tonnes-force, cette presse permet de réaliser le forgeage et de maintenir la pression maximale pendant une durée suffisamment longue qui peut dépasser 10 min. Elle est équipée d'un capteur de force et d'un capteur de déplacement permettant d'enregistrer, au cours du forgeage, la force appliquée et le déplacement du poinçon. La vitesse de forgeage peut varier entre 10 et 150 cm/min. Pour la réalisation de cotyles, la matrice utilisée qui est représentée sur la figure 2a est constituée de la façon suivante :
- une ceinture métallique 1 dont la partie interne cylindrique a un diamètre de 50 mm, un tas inférieur de forme hémisphérique concave 3 qui supporte l'ébauche 5 au début du forgeage et qui est amovible pour faciliter l'extraction de la pièce forgée. La force de forgeage appliquée est mesurée sous cet élément 3. Le poinçon supérieur 7 est un poinçon hémisphérique convexe associé à un tronc de cône pour obtenir la forme interne du cotyle. Il peut se déplacer dans la ceinture métallique 1 à vitesse constante jusqu'à remplissage complet de la matrice.

On porte tout d'abord l'ébauche à la température de forgeage en la chauffant dans une étuve avec ventilation d'air à 140°C pendant 5 heures, l'ébauche étant placée verticalement au contact d'une plaque conductrice à température contrôlée. Après ce chauffage, on l'introduit dans la cavité de la presse de forgeage, comme représenté sur la figure 2a, puis on déplace le poinçon qui est à une température de 20°C jusqu'à ce qu'il vienne en contact avec l'ébauche. On applique alors sur le poinçon une charge de 9,8 tonne-force (soit une pression de 50 MPa) et on déplace ce poinçon à une vitesse de 60 cm/min jusqu'à ce que la cavité de la matrice de forgeage soit complétement remplie par l'ébauche, comme cela est représenté sur la figure 2b. On maintient alors le poinçon 7 dans cette position sous la force maximale de 9,8 tonne-force pendant 2 min, puis on retire le poinçon 7 et le tas hémisphérique 3 pour extraire de la presse la pièce 6 ainsi forgée, comme cela est représenté sur la figure 2c.

Cette pièce présente une bonne sphéricité, elle est exempte de défauts et à les dimensions suivantes :
diamètre extérieur $d_e = 49,8$ mm
diamètre intérieur $d_i = 22,7 \pm 0,1$ mm

On détermine l'orientation des chaînes de polymère dans la pièce forgée ainsi obtenue, par examen optique. Dans ce but, on prélève dans la pièce un échantillon en coupant tout d'abord la pièce en deux à la scie suivant un plan axial et en coupant ensuite chaque demi-pièce au microtome LEITZ 1515 équipé d'un couteau en acier pour obtenir des coupes d'une épaisseur de 25 µm. On place alors les coupes entre deux lames de verre avec un liquide d'indice pour diminuer la réfraction due aux traits de coupe et on les observe par biréfringence en lumière naturelle polarisée linéairement. Cette observation peut être macroscopique sur l'ensemble d'une coupe ou microscopique à l'aide d'un microscope polarisant. On peut ainsi observer :

- les directions des déformations principales qui sont les directions des indices optiques principaux, et
- les valeurs des déformations principales qui dépendent des valeurs des biréfringences optiques principales.

On constate ainsi que les chaînes polymères sont orientées parallèlement à la surface interne du cotyle dans la zone située légèrement en-dessous de la surface de frottement constituée par la partie hémisphérique concave de la pièce forgée.

L'exemple suivant illustre l'amélioration, obtenue grâce au procédé de l'invention, sur la résistance à l'usure et la résistance au fluage de pièces en polyéthylène HD1000 forgées dans le dispositif de la figure 3.

Sur cette figure 3, on voit que la matrice de forgeage comprend une ceinture métallique 11 dont la partie interne cylindrique a un diamètre de 50 mm, un tas inférieur plat 13 qui supporte l'ébauche 15, et un poinçon cylindrique plat 17.

On réalise le forgeage des ébauches comme précédemment dans les conditions suivantes :

- température de forgeage : 140°C
- temperature du poinçon 17 : 20°C
- vitesse de forgeage : 150 cm/min
- temps de maintien du poinçon en position finale : 2 min
- dimensions des ébauches : 50 mm de longueur, 33 mm de diamètre.

On contrôle ensuite la résistance à l'usure et la résistance au fluage des pièces forgées obtenues en réalisant les essais suivants :

## A) ESSAI D'USURE

On utilise pour cet essai un usuromètre de type MARCELIN. Les éprouvettes d'essai ont été extraites et usinées à partir des pièces forgées de manière à tester le polymère sur les surfaces orientées parallèlement à l'écoulement. Ces éprouvettes ont la forme de rondelles d'une épaisseur de 2 ± 0,02 mm et d'un diamètre de 16 ± 0,02 mm qui ont été découpées parallèlement à la direction de forgeage. Les conditions de l'essai sont les suivantes :

On utilise une contreface cylindrique en acier Z 100 C D17, de 30 mm de diamètre et de 0,1 $\mu$m de $r_a$. On entraîne l'arbre cylindrique en rotation à une vitesse de 150 tours par minute, soit 0,4 m/s et on applique une force de 0,5 kg-force pendant 192 h. Les mesures sont effectuées à l'air ambiant à une température de 22°C.

L'évaluation de l'usure est exprimée par la mesure de la corde moyenne (en millimètre) de l'encoche d'usure provoquée. Les résultats obtenus sont donnés dans le tableau 1 qui suit. Dans ce tableau, on a également indiqué les résultats obtenus sur des pièces en polyéthylène HD 1000 qui n'ont pas été forgées.

## TABLEAU 1

| Polyéthylène HD1000 non forgé | Polyéthylène HD1000 forgé |
|---|---|
| 2,77 | 2,46 |
| 2,7 | 2,45 |
| 2,85 | 2,41 |
| moyenne : 2,77 | moyenne : 2,44 |

## B) ESSAI DE FLUAGE

Les essais sont réalisés sur une machine d'essai de fluage en compression selon la norme ASTM D 695-80. Les éprouvettes utilisées ont été usinées à partir des pièces forgées de manière à tester le polymère dans la même direction que le forgeage. Ces éprouvettes sont des cylindres de 7 mm de diamètre et de 10,5 mm de hauteur découpées parallèlement à la direction du forgeage. Les conditions de l'essai de fluage sont les suivantes :

- température 20°C
- contrainte nominale : 28,3 MPa
- durée de l'essai : 48 h.

Les résultats obtenus sont donnés dans le tableau 2 qui suit. Dans ce tableau, on a indiqué également les résultats obtenus sur des échantillons de polyéthylène HD1000 non forgés.

## TABLEAU 2

| Polyéthylène | Déformation instantanée | Fluage après 24h | Fluage après 48h |
|---|---|---|---|
| HD1000 | 12,7% | 18,2% | 19,8% |
| non forgé | 9,2% | 15,9% | 17,8% |
| HD1000 | 13,9% | 14,6% | 15,1% |
| forgé | 13,0% | 14,1% | 15,0% |

Au vu des résultats donnés dans ces deux tableaux, on constate que le forgeage a permis d'améliorer les propriétés de résistance à l'usure et de résistance au fluage des pièces en polyéthylène, ce qui est dû au choix des conditions particulières de forgeage.

**Revendications**

1. Procédé de fabrication d'une pièce en polyoléfine comportant une surface destinée à être en contact avec une autre pièce, dite surface de frottement, caractérisé en ce qu'il comprend les étapes suivantes :

a) porter à une température de forgeage une ébauche en polyoléfine de haut poids moléculaire, dans laquelle le rapport longueur de l'ébauche par rapport à la dimension transversale de l'ébauche est d'environ 0,6 à environ 1,7, la température de forgeage étant située dans la gamme de températures allant d'environ 20°C en-dessous de la température de fusion Tf de ladite polyoléfine à environ 10°C au-dessus de cette température de fusion Tf,

b) disposer l'ébauche portée à ladite température de forgeage dans une matrice de forgeage susceptible d'être obturée par un poinçon déplaçable en translation dans ladite matrice, dans le sens de la longueur de l'ébauche, le poinçon déplaçable et/ou la matrice étant à une température allant de la température ambiante à la température de forgeage,

c) appliquer au poinçon une pression supérieure à la contrainte d'écoulement de ladite polyoléfine à ladite température de forgeage et déplacer ledit poinçon jusqu'à une position finale pour forger ladite ébauche dans ladite matrice, avec un taux de réduction de 0,4 à 0,8 et une vitesse de forgeage de 10 à 150 cm par minute,

d) maintenir le poinçon en position finale pendant une période allant d'environ 90 à environ 150 secondes, et

e) extraire la pièce forgée de ladite matrice.

2. Procédé selon la revendication 1, caractérisé en ce que l'on maintient le poinçon en position finale pendant une durée d'environ 2 min.

3. Procédé selon l'une quelconque des revendications 1 et 2, caractérisé en ce que le poinçon déplaçable est un poinçon hémisphérique.

4. Procédé selon la revendication 3, caractérisé en ce que le poinçon hémisphérique est associé à une matrice cylindrique ayant un fond hémisphérique en regard dudit poinçon.

5. Procédé selon l'une quelconque des revendications 1 à 4, caractérisé en ce que la matrice est réalisée en acier ou en alliage léger.

6. Procédé selon l'une quelconque des revendications 1 à 5, caractérisé en ce que la polyoléfine est du polyéthylène.

7. Procédé selon la revendication 6, caractérisé en ce que la pression appliquée au poinçon déplaçable est de 10 à 80 MPa.

8. Pièce en polyoléfine de haut poid moléculaire, obtenue par le procédé selon l'une quelconque des revendications 1 à 7, de sorte que la structure de la polyoléfine est telle que les chaînes de la polyoléfine sont orientées parallèlement à la surface de frottement, au moins dans la zone de ladite pièce affleurant pratiquement cette surface de frottement, de façon à obtenir une résistance au fluage au bout de 48h équivalant à une déformation par fluage d'au plus 16% dans l'essai de fluage selon la norme ASTM D 695-80.

9. Pièce en polyoléfine selon la revendication 8, caractérisée en ce que la polyoléfine est du polyéthylène.

10. Pièce selon l'une quelconque des revendications 8 et 9, caractérisée en ce que la surface de frottement est hémisphérique.

11. Pièce en polyoléfine selon la revendication 10, caractérisée en ce qu'elle constitue une pièce pour prothèse articulaire.

12. Pièce en polyoléfine selon la revendication 11, caractérisé en ce que la prothèse articulaire est une prothèse de la hanche, du genou ou de l'épaule.

## Claims

1. Process for the production of a polyolefin part having a surface to be in contact with another part, called the friction surface, is characterized in that it comprises the following stages:

a) heating to the shaping temperature a blank of said polyolefin, in which the ratio of the blank length compared with the transverse dimension is between approximately 0.6 and approximately 1.7, the shaping temperature in the range between approximately 20°C below the melting point Tf of said polyolefin and approximately 10°C above said melting point Tf;

b) placing the blank heated to the shaping temperature in a shaping mould which can be sealed by a punch displaceable in translation, in said mould in the heightwise direction of the blank, the displaceable punch and/or matrix being at a temperature between ambient temperature and the shaping temperature;

c) applying to the punch a pressure exceeding the yield stress of said polyolefin at said shaping temperature and displacing said punch up to a final position for shaping said blank in said mould with a reduction rate of 0.4 to 0.8 and a shaping rate of 10 to 150 cm/minute;

d) maintaining the punch in the final position for a period between approximately 90 and approximately 150 seconds to obtain a homogeneous contraction and a good dimensional control of the final part, and

e) extracting the shaped part of the mould.

2. Process according to claim 1, characterized in that the punch is maintained in the final position for a period of approximately 2 minutes.

3. Process according to one of the claims 1 and 2, characterized in that the displaceable punch is a hemispherical punch.

4. Process according to claim 3, characterized in that the hemispherical punch associated with a cylindrical mould having a hemispherical bottom facing the punch.

5. Process according to any one of the claims 1 to 4, characterized in that the mould is made from steel or light alloy.

6. Process according to any one of the claims 1 to 5, characterized in that the polyolefin is polyethylene.

7. Process according to claim 6, characterized in that the pressure applied to the displaceable punch is 10 to 80 MPa.

8. High molecular weight polyolefin part obtained according to any one of the claims 1 to 7, so the structure of the polyolefin is such that the polyolefin chains are oriented parallel to said friction surface, at least in the zone of said part located below or flush with the friction surface, so as to obtain a creep resistance after 48 hours equivalent to a deformation by creep of at the most 16% in the creep test according to ASTM standard D 695-80.

9. Polyolefin part according to claim 8, characterized in that the polyolefin is polyethylene.

10. Part according to one of the claims 8 and 9, characterized in that the friction surface is hemispherical.

11. Polyolefin part according to claim 10, characterized in that it constitutes a part for an articular prosthesis.

12. Polyolefin part according to claim 11, characterized in that the articular prosthesis is a hip, knee or shoulder prosthesis.

## Patentansprüche

1. Verfahren zum Herstellen eines Werkstücks aus Polyolefin, das eine Reibfläche genannte Fläche aufweist, die dazu bestimmt ist, mit einem anderen Werkstück in Berührung zu treten, dadurch gekennzeichnet, daß es die folgenden Schritte umfaßt:

a) ein Rohling aus einem Polyolefin hohen Molekulargewichts, bei welchem das Verhältnis der Länge des Rohlings gegenüber der Querabmessung des Rohlings zwischen etwa 0,6 und etwa 1,7 liegt, wird auf eine Warmbildetemperatur gebracht, die in dem Bereich von Temperaturen liegt, die von etwa 20°C unter der Schmelztemperatur Tf des genannten Polyolefins bis etwa 10% über dieser Schmelztemperatur Tf reichen,

b) Einlegen des auf die genannte Warmbildetemperatur gebrachten Rohlings in eine Warmbildematrize, die dazu geeignet ist, von einem Stempel verschlossen zu werden, der in Längsrichtung in die Matrize in Längsrichtung des Rohlings verschiebbar ist, wobei der verschiebbare Stempel und/oder die Matrize sich auf einer Temperatur befinden, die von der Umgebungstemperatur bis zur Warmbildetemperatur geht,

c) Aufbringen eines Drucks auf den Stempel, der größer ist als die Fließspannung des genannten Polyolefins bei der Warmbildetemperatur, und Verschieben des Stempels bis in eine Endstellung, um den Rohling in der genannten Matrize mit einer Reduktionsrate zwischen 0,4 und 0,8 und einer Formgeschwindigkeit zwischen 10 und 150 cm pro Minute zu verformen,

d) Halten des Stempels in der Endstellung über eine Zeitdauer, die von etwa 90 bis etwa 150 s reicht, und

e) Entnehmen des geformten Werkstücks aus der Matrize.

2. Verfahren nach Anspruch 1, dadurch gekennzeichnet, daß man den Stempel über eine Zeitdauer von etwa 2 min. in der Endstellung hält.

3. Verfahren nach einem der Ansprüche 1 und 2, dadurch gekennzeichnet, daß der verschiebbare Stempel ein halbkugelförmiger Stempel ist.

4. Verfahren nach Anspruch 3, dadurch gekennzeichnet, daß der halbkugelförmige Stempel einer zylindrischen Matrize zugeordnet ist, die gegenüber dem genannten Stempel einen halbkugelförmigen Boden hat.

5. Verfahren nach einem der Ansprüche 1 bis 4, dadurch gekennzeichnet, daß die Matrize aus Stahl oder einer Leichtmetallegierung besteht.

6. Verfahren nach einem der Ansprüche 1 bis 5, dadurch gekennzeichnet, daß das Polyolefin Polyethylen ist.

7. Verfahren nach Anspruch 6, dadurch gekennzeichnet, daß der auf den verschiebbaren Stempel aufgebrachte Druck zwischen 10 und 80 MPa beträgt.

8. Werkstück aus Polyolefin hohen Molekulargewichts, das man nach dem Verfahren nach einem der Ansprüche 1 bis 7 in der Weise erhält, daß die Struktur des Polyolefins eine solche ist, daß die Ketten des Polyolefins parallel zur Reibfläche orientiert sind, wenigstens in dem Bereich der genannten Fläche, die praktisch mit dieser Reibfläche fluchtet, um einen Fließwiderstand am Ende von 48 h zu erhalten, der einer Verformung durch Fließen von höchstens 16% im Fließversuch nach der ASTM-Norm D 695–80 entspricht.

9. Werkstück aus Polyolefin nach Anspruch 8, dadurch gekennzeichnet, daß das Polyolefin Polyethylen ist.

10. Werkstück nach einem der Ansprüche 8 und 9, dadurch gekennzeichnet, daß die Reibfläche halbkugelförmig ist.

11. Werkstück aus Polyolefin nach Anspruch 10, dadurch gekennzeichnet, daß es ein Teil für eine Gelenkprothese bildet.

12. Werkstück aus Polyolefin nach Anspruch 11, dadurch gekennzeichnet, daß die Gelenkprothese eine Hüft-, eine Knie- oder eine Schulterprothese ist.

EP 0 196 946 B1

FIG.1

FIG.2a

FIG.2b

FIG.2c

FIG.3